# EUROPEAN PATENT SPECIFICATION

(11) **EP 2 704 731 B1**
(45) Date of publication and mention of the grant of the patent: **13.11.2019**
(21) Application number: 12780015.9
(22) Date of filing: 02.05.2012
(51) Int. Cl.: A61K 35/12, A61P 17/00, A61P 35/00, A61K 35/57

(54) **AVIAN-BASED TREATMENT**
BEHANDLUNG MIT VOGELEXTRAKTEN
TRAITEMENT D'ORIGINE AVIAIRE

(30) Priority: 02.05.2011 AU 2011901620; 13.02.2012 AU 2012900513
(43) Date of publication of application: 12.03.2014
(73) Proprietor: Cocky Smart Pty Ltd, Crossman, WA 6390 (AU)
(72) Inventor: CHAMBERLAIN, John, CROSSMAN Western Australia 6390 (AU)
(74) Representative: AWA Sweden AB
(86) International application number: PCT/AU2012/000460
(87) International publication number: WO 2012/149601

(56) References cited:
- WO-A1-2008/019452
- WO-A1-2008/019453
- WO-A1-2008/019453
- WO-A2-2009/022842
- WO-A2-2009/057975
- US-A- 3 970 614
- US-A- 4 818 520
- RIGANO, L. ET AL.: 'Novel Retinol-Like Actives from Parrot Feathers' IFSCC vol. 11, no. 8, 2008, pages 323 - 330, XP008110401
- BURTT JR, E.H. ET AL.: 'Colourful Parrot Feathers Resist Bacterial Degradation' BIOLOGY LETTERS. vol. 7, no. 2, 23 April 2011, pages 214 - 216, XP055134466

## Description

### Field of the Invention

The present invention relates to a composition and a kit for use in the treatment or prevention of proliferative skin disorders, cancers and pre-cancers.

### Background of the Invention

Proliferative disorders affect many millions of people worldwide and are caused by an over-response of certain cells (often those associated with immune response) to a stimulus; or by the overgrowth of invasive cells; or an abnormal proliferation of cells constituting cancer.

Many of the proliferative disorders suffered are proliferative skin disorders. The best known and most severe form of proliferative skin disorder is skin cancer.

One characteristic of numerous proliferative skin disorders is epidermal hyperplasia. Epidermal hyperplasia is an abnormal increase in the number of normal cells in normal arrangement in epidermal tissue. Often the abnormally increased cells are keratinocyte cells. It is postulated that epidermal hyperplasia involves a complex multi-cellular inflammatory event.

An example of a severe proliferative skin disorder is psoriasis, a chronic, inflammatory, hyperproliferative skin condition that affects approximately 2% of the general population. Typical symptoms of psoriasis include skin lesions, redness, inflammation, or patches of skin that become dry, red, covered with silvery scales, cracked, and/or painful.

Current psoriasis treatment suffers from a number of drawbacks. For example, many of the currently-available, topical anti-psoriatic agents irritate the skin, cannot be used for extended durations, and/or lead to aggressive recurrence of the psoriatic condition if treatment is terminated abruptly. Anti-inflammatory agents, although capable of alleviating certain symptoms, do not cure the underlying disease. Another current treatment option, photochemotherapy, can lead to squamous-cell and melanoma skin cancer.

Another range of proliferative skin disorders is grouped under the term 'eczema'. The severity of the disease can vary from mild forms (dry, hot and itchy skin) to more severe forms, where the skin can become broken and raw, and may bleed. The underlying causes of eczema are many and varied depending on the type of eczema. Although several types of eczema are well characterised - atopic eczema, allergic contact dermatitis, infantile seborrhoeic eczema, adult seborrhoeic eczema, varicose eczema and discoid eczema - the underlying factor is the immune system responding to an allergic stimulus by proliferating protective cells to isolate the areas and/or negate the allergen involved.

Although there is currently no known cure for eczema, a number of treatments have been devised with a view to minimising the discomfort and distress associated with eczema.

A further common proliferative skin disorder is the proliferation of skin cells that can occur during the process of scarring. Abnormal concentrations of cells, particularly fibroblasts, can lead to areas of fibrosis. This is especially the case for example in keloid scarring. Keloid or keloid scars are hypertrophic as a result of uncontrolled cell growth and extend beyond the boundaries of a wound. They typically appear following surgery or injury, but can also appear spontaneously or as a result of some slight inflammation, such as an acne pimple.

Cancer is characterized by unregulated proliferation of cells in the body, and can arise in any body tissue. Cancerous cells propagate from a single cell and multiply without control to develop into tumour tissues. These cancerous cells can invade nearby tissues and can spread through the bloodstream and lymphatic system to other parts of the body (a process referred to as metastasis). Cancer is a serious illness affecting many people every year and the mortality rate for the most common cancers still remains high.

One of the more common types of cancer is the proliferative skin disorder known as skin cancer. The three major types of skin cancer are basal cell carcinoma, squamous cell carcinoma, and melanoma. Basal cell and squamous cell carcinomas can cause substantial illness and, when left untreated, can cause considerable damage and disfigurement. The chances for curing this form of cancer is favourable, but only if detected and treated early.

Malignant melanoma causes more than 75% of all deaths from skin cancer. This is the most serious form of skin cancer and can spread to other parts of the body quickly, after which it is very difficult to treat. However, when detected in its earliest stages and treated properly it is also highly curable.

There are also a number of precancerous conditions and skin lesions (such as actinic keratoses or solar keratoses; or dysplasic nevus or atypical mole) which are preferably treated before they develop into a cancerous lesion.

Other cancers include solid tumours such as carcinomas (e.g. most breast, prostate, lung, pancreatic and colon cancers), sarcomas (e.g. cancers deriving from connective tissue such as bone, cartilage, fat and nerve cells), lymphomas (deriving from lymphatic or blood cells), germ cell tumours (cancers derived from pluripotent cells of the testes or ovaries) and balstomas (derived from immature "precursor" cells or embryonic tissue). There are also cancers such as leukaemias which, like lymphomas derive from lymphatic or blood cells, but which do not form solid tumours.

Current treatments for cancers, including skin cancers, generally fall under the categories of surgery, radiation therapy and/or chemotherapy.

Chemotherapy drugs are subdivided into specific classes such as alkylating agents, antimetabolites, anthracyclines, and topoisomerase inhibitors. They are usually given by IV infusion, but can be given orally. Chemotherapy is often contrasted with targeted (biologic) therapy or immunotherapy, which attack specific parts of specific cells instead of all dividing cells.

Chemotherapy has become a standard treatment method to control, but usually not cure, most types of cancer, including melanoma. Chemotherapeutic drugs used to treat melanoma include dacarbazine, temozolomide, paclitaxel, cisplatin, carmustine, fotemustine, vindesine, vincristine, and bleomycin. Often combinations of chemotherapy agents are used in the treatment of melanoma -- the CVD (cisplatin, vincristine and dacarbazine) and BVLD (bleomycin, vincristine, lomustine and dacarbazine) regimens are examples of this.

Chemotherapy generally is only effective in treating melanoma in 15-20% of patients. Typically, it works for less than a year and has no effect on survival time. Chemotherapy can also cause mild to severe side effects due to the chemotherapeutic drugs impacting cells that normally grow quickly, such as hair, blood, stomach, and intestinal cells. Common side effects include nausea, vomiting, hair loss, fatigue, anaemia, muscle/nerve pain and a variety of other symptoms.

Given the unwanted side effects of most current chemotherapy drugs, in recent years considerable emphasis has been given to the development of new chemotherapeutic agents, especially those with lower side effects.

Accordingly, there is a need for novel pharmaceutical compositions containing bioactive substances which are able to treat or at least ameliorate proliferative conditions, including proliferative skin conditions such as eczema, psoriasis, scarring, pre-cancer and cancer.

### Summary of the Invention

In one embodiment, the invention provides a composition comprising nicotinamide 1-10%, 2,4-ditert butyl phenol 1-10%, palmitic acid 5-40%, oleic acid 2-20%, and squalene 5-30% together with a pharmaceutically acceptable carrier and/or diluent for use in the treatment or prevention of cancers or pre-cancers selected from the group consisting of: skin cancer (including basal cell carcinoma, squamous carcinoma or melanoma), precancerous skin lesion (such as actinic or solar keratosis), carcinoma (including breast, prostate, lung, pancreatic and colon cancers), sarcoma, lymphoma, germ cell tumour, blastoma, and leukaemia.

According to a further embodiment, the invention provides a kit for use in the treatment or prevention of a cancer or pre-cancer comprising: a composition comprising nicotinamide 1-10%, 2,4-ditert butyl phenol 1-10%, palmitic acid 5-40%, oleic acid 2-20%, and/or squalene 5-30% and a pharmaceutically acceptable carrier and/or diluent; and instructions regarding the administration of the composition for the treatment or prevention of a cancer or pre-cancer, wherein the cancer or pre-cancer is selected from the group consisting of: skin cancer (including basal cell carcinoma, squamous carcinoma or melanoma), precancerous skin lesion (such as actinic or solar keratosis), carcinoma (including breast, prostate, lung, pancreatic and colon cancers), sarcoma, lymphoma, germ cell tumour, blastoma, and leukaemia.

An extract (hereafter known as EP-2) from the feathers of birds in a therapeutically effective concentration together with a pharmaceutically acceptable carrier for the treatment or prevention of proliferative skin disorders is disclosed.

A method for the treatment or prevention of a proliferative skin disorder, wherein the method comprises administering to a subject in need thereof a composition comprising a therapeutically effective amount of EP-2 together with a pharmaceutically acceptable carrier and/or diluent is disclosed. The proliferative skin disorder may be psoriasis, eczema or scarring.

A method for the treatment or prevention of cancer, wherein the method comprises administering to a subject in need thereof a composition comprising a therapeutically effective amount of EP-2 together with a pharmaceutically acceptable carrier and/or diluents is disclosed. The cancer may be cancer of the skin and even more preferred melanoma. Alternatively, the cancer may be a tumour or solid cancer.

It is further disclosed that the condition to be treated or prevented may be a precancerous lesion and the method comprises administering to a subject in need thereof a composition comprising a therapeutically effective amount of EP-2 together with a pharmaceutically acceptable carrier and/or diluent.

### Figures

Figure 1 shows a graph of the effect of EP-2 or 10 ng/mL TPP (Triphenyl phosphate) on the growth of A2058 melanoma cells over 9 days.
Figure 2 shows a graph of the effect of pooled solvent vehicle (used to extract EP-2), EP-2 or TPP on the growth of A2058 melanoma cells. The graph further shows that the effect of EP-2 on cell growth was reversible after removal of the extract.
Figure 3 shows a graph of the effect of a 1/5 dilution of EP-2 on the growth of A2058 melanoma cells or HEK293 non-cancerous human embryonic kidney cells. The diluted EP-2 had little effect on the growth of A2058 cells but was moderately effective in retarding the growth of HEK293 cells.
Figures 4a and 4b provide graphs of the number of Melanoma A2058 cells (× 106) grown in the presence or absence of Bird Extract (BE) for 1-3 days.
Figure 5 is a graph of Melanoma cell counts showing the effect of varying concentrations of BE on cells. Cells grown in the presence (Concentration 1 (high), Concentration 2 (10 fold dilution)) or absence (control) of BE for one day.
Figure 6 is a graph of Melanoma cell growth curves showing the rate of cell proliferation when Melanoma cells (A2058 cells) were grown in the presence of BE at high and low concentrations over five days relative to Melanoma cells without BE (controls).
Figure 7 is a graph of non-cancerous Human Embryonic Kidney (HEK293) cellular growth curves showing the rate of cell proliferation when HEK293 cells were grown in the presence of BE at high and low concentrations over five days relative to HEK293 cells without BE (controls).
Figure 8 is a graph of Breast Cancer cell (MCF-7) growth curves showing the rate of cell proliferation when Breast cancer cells were grown in the presence of BE at high and low concentrations over five days relative to Breast Cancer cells without BE (controls).
Figure 9 is DAPI nuclear stained images of Melanoma cells plus Bird Extract 200x magnification; ii: Melanoma cells plus Bird Extract 400x magnification; iii: Melanoma cells no extract 200X magnification; iv: Melanoma cells no extract 400x magnification.
Figure 10 shows cells after Bird Extract. The morphology of the cells indicates that they are undergoing cell death.
Figure 11 shows Melanoma cells without (i) or with (ii) Bird Extract; Haemotoxylin and Eosin stain. i=Melanoma cells at 200x Magnification; ii=Melanoma cells plus Bird Extract at 400x Magnification.
Figure 12 is a graph of the percentage of LDH released in BE treated cells relative to untreated controls as a measure of toxicity of BE on Melanoma Cells (A2058) and Embryonic Kidney Cells (HEK293) at various concentrations of BE(C1 low) and C2 (high).
Figure 13 is a graph of the gene expression profiles of Melanoma cells (A2058) and Embryonic Kidney cells (HEK293) show changes in gene expression after treatment with Bird Extract after 1 or 3 days of treatment.
Figure 14 is a graph of changes in gene expression of Melanoma cells (A2058) and Embryonic Kidney cells (HEK293) exposed to Bird Extract for 1 day, with expression levels normalized to GAPDH.
Figure 15 is a graph of Melanoma Cancer (A2058) cell growth curves showing the rate of cell proliferation when the cells were grown in the presence of Synthetic Mixture at high and low concentrations over three days relative to Melanoma Cancer cells without Synthetic Mixture (control media).
Figure 16 is a graph of Breast Cancer (MCF-7) cell growth curves showing the rate of cell proliferation when the cells were grown in the presence of Synthetic Mixture at high and low concentrations over three days relative to Breast Cancer cells without Synthetic Mixture (control media).
Figure 17 is a graph of Normal Embryonic Kidney (HEK293) cell growth curves showing the rate of cell proliferation when the cells were grown in the presence of Synthetic Mixture at high and low concentrations over three days relative to normal Embryonic Kidney cells without Synthetic Mixture (control media).

### Detailed Description of the Invention

### General

Those skilled in the art will appreciate that the invention described herein is susceptible to variations and modifications other than those specifically described herein and for the purpose of completeness these are included. The invention also includes all of the steps, features, compositions and compounds referred to or indicated in the specification, individually or collectively and any and all combinations or any two or more of the steps or features.

Functionally equivalent products, compositions and methods whether occurring naturally from birds or manufactured chemically to replicate the components of the avian extract are disclosed herein.

Throughout this specification, unless the context requires otherwise, the words "comprise", "comprises" or "comprising", will be understood to imply the inclusion of a stated integer or group of integers but not the exclusion of any other integer or group of integers.

Definitions for selected terms used herein may be found within the detailed description of the invention and apply throughout. Unless otherwise defined, all other scientific and technical terms used herein have the same meaning as commonly understood to one of ordinary skill in the art to which the invention belongs.

### Detailed Description of the Preferred Embodiments

It is disclosed that an extract from the skin and feathers of birds can be used to treat various proliferative skin disorders and cancer, which is also a disease of over proliferation of cells. It is further disclosed that a composition comprising an avian extract (hereafter known as EP-2) from the skin and feathers of birds in a therapeutically effective concentration together with a pharmaceutically acceptable carrier may be used in the treatment or prevention of proliferative skin disorders, cancers and pre-cancers.

Reference to 'feathers' may be taken to mean 'feathers and skin' together, or 'feathers' or 'skin' individually. It is believed that the active components of the disclosed extract are provided on both the skin and the feathers of the bird and may spread from one area to another (e.g. from the skin of the bird along the feather shaft). The extract may be taken from whichever region of the bird is most convenient to collect from.

Whilst a similar extract has been disclosed by Chamberlain in WO 2008/019453 "Avian-based Insect Repellent", this extract was used solely as an insect repellent. The extract disclosed in WO 2008/019453 may be used in the treatment or prevention of proliferative skin disorders such as skin cancer, precancerous lesions, eczema, psoriasis, scarring or other proliferative disorders and other cancers and precancers such as solid tumours and leukaemia. Further, WO 2008/019452 relates to insect repellents comprising an effective amount of a phosphorous containing compound of the formula (RO)₃P=Oₓ or (RO)₂P=Oₓ(OH), alone or in synergistic combination with hydrocarbons, alkyl esters or dicarboxylic acids.

There is disclosed a method for treating or preventing proliferative skin conditions, cancers or pre-cancers comprising the step of: administering to a subject in need thereof a composition comprising avian extract EP-2 in a therapeutically effective concentration together with a pharmaceutically acceptable carrier and/or diluent.

It is disclosed that the avian-based EP-2 extract may be obtained from birds of the order Psittaciformes (parrots). Suitable examples of such birds include Australian native parrots, such as the sulphur-crested cockatoo (Cacatua galerita), the galah (Cacatua roseicapilla), and the Major Mitchell cockatoo (Cacatua leadbeateri leadbeateri). It is further disclosed that the avian extract EP-2 may be obtained from bird feathers, in particular tail feathers. It is further disclosed that the EP-2 may be obtained from the tail feathers of the sulphur-crested cockatoo.

The term "extract" as used herein refers to one or more compounds, typically in concentrated form, obtained by treating a material from which the extract is isolated with a solvent, after which the solvent is preferably removed. The term "extract" will also be understood to encompass one or more compounds obtained by subjecting a primary extract to subsequent purification processes known to those skilled in the art.

The avian-based extract may be extracted from bird feathers, in particular tail feathers, by treating the feathers with any one or more of a range of organic solvents. Suitable examples of organic solvents include, hydrocarbons (for example butane, pentane, hexane, heptane and octane); halogenated hydrocarbons (for example, methylene chloride, chloroform, trichloroethylene, carbon tetrachloride, trichloroethane, or trifluormethane); ethers (for example n-hexyl ether, methyl phenyl ether, ethyl phenyl ether and ethyl benzyl ether); ketones and aldehydes (for example acetone, acetonyl-acetone, benzaldehyde, acetophenone); and other oils such as vegetable or mineral oils. The organic solvent can then be removed by techniques well understood to those skilled in the art, leaving a neat concentrate of EP-2.

Alternatively, EP-2 may be extracted derived from the tail feathers of a bird by aqueous extraction, any other form of solvent extraction or physical dusting.

The avian-based extract EP-s may be extracted into 95% organic solvent. Solvents which may be used include Ethanol 95%, Dimethyl Sulfoxide (DMSO), or Dimethyl Formamide (DMF). Further, ethanol 95% may be used as the organic solvent.

The avian extract EP-2 may also be replicated in a synthetic form by combining two or more of the components of naturally occurring EP-2 to produce a Synthetic Mixture. This could be in the form of a single entity selected from the components of the avian extract, or a combination of two or more components. Preferably, the two or more components are in a synergistic relationship. The term "extract" will also be understood to mean any replicate formulation composed of one of more of the components that can be isolated from the avian extract or manufactured by chemical synthesis to resemble one of more of the compounds found in the extract. Thus, the term "extract" can be understood to mean the Synthetic Mixture.

For example the compounds TPP, Norpinene, Palmitic acid, Oleic acid, Benzoic acid, Napthalene, Nonanoinc acid, derivatives of Butylphenol, esters such as Hexadecanyl ester, phalates such as Iso- or Butyl- derivatives and Dioctadecyl phosphates have all been isolated as part of the EP-2 extract and may form part of the Synthetic Mixture. Formulations that comprise combinations of one or more chemical equivalents which may be available commercially and therefore economically of advantage to use to deliver an equivalent therapeutically effective dose are also disclosed.

The Synthetic Mixture preferably comprises the following compounds: nicotinamide 1-10%, 2,4-ditert butyl phenol 1-10%, palmitic acid 5-40%, oleic acid 2-20%, and/or squalene 5-30%. Alternatively, the Synthetic Mixture may comprise the following compounds: nicotinamide at about 5.9%, 2,4-ditert butyl phenol at about 2.5%, palmitic acid at about 19.8%, oleic acid at about 7.6%, and/or squalene at about 12.1%

The Synthetic Mixture may optionally also include BHT-aldehyde at about 0.05-2% and/or cholestenol at about 1-10%. Alternatively, the Synthetic Mixture may optionally comprise BHT-aldehyde at about 0.2% and/or cholestenol at about 4.0%.

The Synthetic Mixture can be dissolved in methanol as the solvent, or in water as the solvent to generate a solution for use as an extract.

It is disclosed that the avian extract EP-2 may be further processed into a powder form by, for example, air drying or freeze drying to remove moisture or the extraction solvent. Such dried compositions may be reconstituted in a suitable carrier or diluents for later application to the skin or cancer. It is disclosed that the extracted EP-2 may be dried under vacuum until less than 1% moisture remains. The extract may then be reconstituted using an organic solvent, for example Ethanol 95%, Dimethyl Sulfoxide (DMSO) or Dimethyl Formamide (DMF) which will aid in delivering an effective therapeutic dose. For example, DMSO is well known to those skilled in the art to aid in the penetration of topical therapeutics.

It is disclosed that the composition EP-2 may be used to treat or prevent a wide range of proliferative skin disorders, cancers and pre-cancers.

Generally, the terms "treat", "treating" and "treatment" and derivatives used herein have the meaning to affect a subject, tissue or cell to produce a desired pharmacological and/or physiological effect. The treatment may be therapeutic in terms of: preventing progression of the proliferative skin disorder, pre-cancer or cancer; relieving or ameliorating the effects of the disorder, pre-cancer or cancer; or causing a partial or complete cure and/or regression of the proliferative skin disorder, pre-cancer or cancer.

"Preventing" or "prevention" and derivative terms relate to the partial or complete prevention of development of a proliferative skin disorder or cancer or its symptoms in a subject who: has not yet been diagnosed with a disorder or cancer; has the disorder or cancer in some but not all of their organs, such as skin, and wishes to prevent it developing on new regions; has had the disorder or cancer and may be in remission and wishes to prevent re-occurrence; or has been diagnosed as being at risk of developing a proliferative skin disorder or cancer.

For example, it is disclosed that the extract may be used to treat a proliferative skin condition chosen from the following non-exhaustive list: eczema (including atopic eczema, allergic contact dermatitis, infantile seborrhoeic eczema, adult seborrhoeic eczema, varicose eczema and discoid eczema), psoriasis (including chronic plaque syndrome, pustular psoriasis, erythrodermic psoriasis or nail psoriasis), and scarring including keloid scarring. The composition may be used to treat or prevent psoriasis, or keloid scarring.

It is disclosed that the EP-2 avian extract may be used to prevent the development of a proliferative skin disorder in a subject who has not been diagnosed with a proliferative skin disorder. Such subjects may, however, have been diagnosed as being at risk or susceptible to proliferative skin disorders due to some other prevailing medical condition or event rendering them more susceptible. Alternatively, the subject may not be currently suffering from a proliferative skin disorder, but may suffer from recurring bouts of such disorders and may wish to prevent the recurrence of another bout.

There is also disclosed an avian-based extract (EP-2) for the use in treatment of cancer, most preferably, skin cancer. Alternatively, the avian extract EP-2 may be used to treat pre-cancerous conditions such as carcinoma *in situ*, dysplasia, Barrett's oesophagus, Cervical intraepithelial neoplasia, precancerous skin lesions, actinic or solar keratosis, and may be used to prevent such conditions subsequently developing into malignant cancers. The avian extract may be used to treat or prevent malignant or non-malignant/benign cancers and tumours.

Alternatively, the cancer is a cancer selected from the group consisting of: sarcomas, carcinomas and other solid tumour cancers, including germ line tumours, tumours of the central nervous system, breast cancer, prostate cancer, skin cancer, cervical cancer, uterine cancer, lung cancer, ovarian cancer, testicular cancer, thyroid cancer, astrocytoma, glioma, pancreatic cancer, stomach cancer, liver cancer, colon cancer, renal cancer, bladder cancer, oesophageal cancer, cancer of the larynx, cancer of the parotid, cancer of the biliary tract, rectal cancer, endometrial cancer, adenocarcinomas, small cell carcinomas, blastomas (including neuroblastomas and hepatoblastomas), mesotheliomas, adrenocortical carcinomas, epithelial carcinomas, desmoid tumours, desmoplastic small round cell tumours, endocrine tumours, bone cancers (such as osteoma, osteoid osteoma, osteochondroma, osteoblastoma, enchondroma, giant cell tumour of bone, aneurysmal bone cyst, fibrous dysplasia of bone, osteosarcoma, chondrosarcoma, Ewing's sarcoma, fibrosarcoma), hepatocellular carcinomas, non-rhabdomyosarcome soft tissue sarcomas, osteosarcomas, peripheral primative neuroectodermal tumours, retinoblastomas, rhabdomyosarcomas, Wilms tumours, lymphoma; and leukaemia. The cancer may be a malignant cancer or a benign cancer.

The cancer may alternatively be a sarcomas (e.g. cancers deriving from connective tissue such as bone, cartilage, fat and nerve cells),

More preferably the cancer is a type of skin cancer. Most preferably the skin cancer is a type of basal cell carcinoma, squamous carcinoma or melanoma.

In one embodiment the subject has not been diagnosed with cancer but may be diagnosed as being at risk of developing cancer. For example, the subject may suffer with pre-cancerous conditions such as carcinoma in situ, dysplasia, Barrett's oesophagus, Cervical intraepithelial neoplasia, precancerous skin lesions, actinic or solar keratosis and may wish to treat the said pre-cancerous condition and/or prevent the subsequent onset of malignant or benign cancer.

It is disclosed that the subject to be administered the composition may be a warm blooded vertebrate, for example a human, a companion animal such as dog or cat, domestic animal such as horse, cattle and sheep, or zoo animal such as non-human primate, canids, bovids, felids and ungulates. Alternatively, the subject in need of treatment may be avian. It is disclosed that the subject may be a human.

A composition comprising a therapeutically effective amount of avian extract EP-2 and one or more pharmaceutically acceptable additives, excipients carriers and/or diluents is disclosed. Further, the term "therapeutically effective amount" means an amount of EP-2 effective to yield a desired therapeutic response, for example to prevent or treat a proliferative skin disorder such as psoriasis, eczema, scarring, pre-cancerous conditions and/or cancer. The specific therapeutically effective amount will of course vary with such factors as the particular condition being treated, the physical condition and clinical history of the subject, the type of animal being treated, the duration of the treatment, the nature of concurrent therapy (if any), the specific formulations employed and the structure of the composition.

The term "pharmaceutically", "physiologically", or "veterinary acceptable" as used herein refers to pharmaceutically active agents, physiologically active agents, veterinary active agents, or inert ingredients which are suitable for use in contact with the skin of animals, including humans, without undue toxicity, incompatibility, instability, irritation, allergic response, commensurate with a reasonable benefit/risk ratio.

Additives, excipients carriers and diluents for use in the compositions of the present invention include, water, saline, ethanol, dextrose, glycerol, glycerol and polyhydric alcohols, milk protein, vitamins, animal and vegetable oils, polyethylene glycols, lactose, dextrose, sucrose sorbitol, mannitol and other sugars, starches, gum acacia, calcium phosphates, alginate, tragacanth, gelatine, calcium silicate, cellulose and its derivatives such as microcrystalline cellulose and methyl cellulose, polyvinylpyrrolidone, water syrup, methyl and propylhydroxybenzoates, talc, magnesium carbonate, titanium dioxide, magnesium stearate and mineral oil or combinations thereof.

The formulations can additionally include lubricating agents, dispersion media, pH buffering agents, wetting agents, emulsifying and suspending agents, solvents, preserving agents, sweetening agents or flavouring agents, antifoaming agents, polymers, antioxidants, chelating agents, viscomodulators, tonicifiers, flavorants, colorants, odorants, opacifiers, suspending agents, binders, fillers, plasticizers, lubricants, absorption-promoting agents and mixtures thereof. Preservatives include antimicrobial, antibacterial and antifungal agents, anti-oxidants, chelating agents and inert gases. The particular selection of constituent that can be included in the compositions described herein will generally depend on the type of preparation.

It is disclosed that the composition may be formulated to enable ready location and retention of the active ingredient(s) of the EP-2 extract in the area required to deliver the therapeutic effect while at the same time not significantly interfering with EP-2's efficacy. It will be understood, therefore, that the therapeutically effective composition may be formulated differently based on the area of treatment and how the composition is to be applied.

There are a range of reference sources for the development of cosmetic and pharmaceutical compositions which may be referred to by the skilled person when developing formulations comprising EP-2, such as "A Formulary of Cosmetic Preparations; Volume 1 - Decorative Cosmetics", Hunting L.L. (2003); "A Formulary of Cosmetic Preparations; Volume 2 - Creams, Lotions and Milks", Hunting L.L. (2004) and "Remington's Pharmaceutical Sciences", 21st Edition (2009), Mack Publishing Company, Easton, Pennsylvania, USA, all the contents of which are incorporated herein. Other suitable guidebooks include Cosmetics and Toiletries Magazine, Vol. 111 (March, 1996); Formulary: Ideas for Personal Care; Croda, Inc, Parsippany, N.J. (1993); and Cosmeticon: Cosmetic Formulary, BASF.

It is disclosed that an EP-2 composition may be administered using standard procedures, for example, topically, parenterally, intraorbitally, ophthalmically, intraventricularly, intracranially, intracapsularly, intraspinally, intracisternally, intraperitoneally, buccally, rectally, vaginally, intranasally, orally or by aerosol administration and/or inhalation spray or via an implanted reservoir.

It is disclosed that the extract may be applied topically for the treatment of proliferative skin disorders including scarring, pre-cancerous conditions and skin cancer. The topical application may be in the form of directly laying or spreading the composition comprising the extract on the area of the proliferative skin disorder (or at least near or adjacent the proliferative skin disorder) using an applicator, such as a brush or a sponge, by spraying the composition directly onto the area, or by rubbing the composition onto the area. Alternatively, the EP-2 may be topically applied to an area of skin which does not yet suffer from a proliferative skin disorder.

Alternatively, it is disclosed that the extract may be administered internally for the treatment of pre-cancerous conditions and cancer. For example, the extract may be delivered by oral routes in the form of a tablet, capsule, liquid dose or powder; by injection e.g. into the blood stream, muscle tissue or directly into an organ such as the eye; or by inhalation.

Pharmaceutically acceptable carriers, adjuvants and vehicles that may be used in topical, oral parenteral or inhaled compositions may include ion exchangers; alumina; aluminum stearate; lecithin; self-emulsifying drug delivery systems such as alpha- tocopherol polyethylene glycol 1000 succinate, or other similar polymeric delivery matrices or systems such as nanoparticles; serum proteins such as human serum albumin; buffer substances such as phosphates; glycine; sorbic acid; potassium sorbate; partial glyceride mixtures of saturated vegetable fatty acids; water; salts or electrolytes, such as protamine sulfate, disodium hydrogen phosphate, potassium hydrogen phosphate, sodium chloride, zinc salts; colloidal silica; magnesium trisilicate; polyvinyl pyrrolidone; cellulose-based substances; polyethylene glycol; sodium carboxymethylcellulose; polyacrylates; polyethylene-polyoxypropylene-block polymers; and wool fat.

It is disclosed that the EP-2 composition may further contain one or more additives, provided that they do not detrimentally affect the therapeutic effect afforded by the avian-based extract. The additive may be a colourant. Further, the additive may be a preservative such as a mould inhibitor or an anti-oxidant, a fragrance, or a stabiliser. In another embodiment the additive is a surfactant or an absorption-promoting agent such as dimethyl sulphoxide (DMSO). The additional additives may also be agents that render the composition an emulsion, a microemulsion or a nano-emulsion.

More specifically and by way of example, a suitable vehicle for topical administration of EP-2 to the skin surface might include DMSO (dimethyl sulfoxide), ethanol, acetone, phosphatidyl choline and isopropanol gels. Additional additives may include a synergistic additive such as vitamin E to nutrify the skin, lanolin or aloe vera to soothe the skin. Other suitable additives would be known to those skilled in the art of pharmaceutical or cosmetic formulation.

The composition may be adapted for topical application and may be in a form selected from the group comprising cosmetically acceptable liquids, creams, oils, lotions, ointments, gels, roll-on liquids, skin patches, sprays, glass bead dressings, synthetic polymer dressings impregnated with basic milk factors, solids, conventional cosmetic night creams, foundation creams, suntan lotions, hand lotions, make-up, make-up bases and masks. It is disclosed that except insofar as any conventional medium or agent is incompatible with the active ingredient, use thereof in the cosmetic compositions may be contemplated. Compositions of the present invention adapted for topical delivery will desirably possess bioadhesive or mucoadhesive properties.

It is disclosed that the extract may be delivered parenterally, preferably by injection, for example subcutaneously or intramuscularly. However, delivery may also be intra-arterially, intraperitoneally, intracavital, or intranasally for use in the treatment of proliferative skin disorders, pre-cancerous conditions and cancers.

Pharmaceutically acceptable carriers, adjuvants and vehicles that may be used in injection compositions and may include ion exchangers; alumina; aluminium stearate; lecithin; self-emulsifying drug delivery systems (SEDDS) such as alpha-tocopherol polyethyleneglycol 1000 succinate, or other similar polymeric delivery matrices or systems such as nanoparticles; serum proteins such as human serum albumin; buffer substances such as phosphates; glycine; sorbic acid; potassium sorbate; partial glyceride mixtures of saturated vegetable fatty acids; water; salts or electrolytes, such as protamine sulfate, disodium hydrogen phosphate, potassium hydrogen phosphate, sodium chloride, zinc salts; colloidal silica; magnesium trisilicate; polyvinyl pyrrolidone; cellulose-based substances.

The formulation may be in the form of a sterile injectable preparation, for example, as a sterile injectable suspension for the use in treatment of proliferative skin conditions, pre-cancers and cancers. This suspension may be formulated according to techniques known in the art using suitable dispersing agents, surfactants, and suspending agents (e.g. Tween 80). The sterile injectable preparation may also be a sterile injectable solution or suspension in a non-toxic parenterally- acceptable diluent or solvent (e.g. 1, 2-propanediol). Acceptable vehicles and solvents may include mannitol, water, Ringers solution and isotonic sodium chloride solution with buffer. Furthermore, sterile, fixed oils may be employed as a solvent or a suspending medium. For this purpose, any bland fixed oil may be employed including mono- or diglycerides. Fatty acids, such as oleic acid and its glyceride derivatives, and natural pharmaceutically acceptable oils, such as polyoxyethylated olive oil or castor oil, may also be used in the preparation of injectables.

It is disclosed that if the formulation is to be delivered topically, a range of different formulations may be developed to provide the avian extract EP-2 to the skin. For example, the EP-2 may be delivered as a spray with ethanol or propylene glycol; a lotion with cetomacrogol lotion, aminobenzoic acid lotion or alcohol; a gel with poloxamer gel 8E, poloxamer gel 8C, chlorhexidine gel or Lutrol® F127; an ointment with liquid paraffin or white soft paraffin; or a buffered cream with emulsifying ointment, glycerol, cetomacrogol emulsifying wax 15 or propylene glycol.

Alternative formulations include gels, liquids, liquid solvents, dips, pastes, sprays, aerosols, and other solid formulations such as, for example, a wax-based solid. Other compositions can be formulated by those of ordinary skill in the art.

For example, it is disclosed that the EP-2 extract may be formulated into:
- a spray for use in the treatment of proliferative skin disorders including scarring, pre-cancerous conditions and skin cancer, comprising EP-2 extract together with ethanol 95%, 75%, propylene glycol 5% and water to 100%; formulated in a fashion that will be familiar to those skilled in the art or as described in Remington, 2009. The pH and exact concentration of the various components of the composition are adjusted according to routine skills in the art.
- a lotion for use in the treatment of proliferative skin disorders including scarring, pre-cancerous conditions and skin cancer, comprising EP-2 extract together with any one of the following: cetomacrogol lotion, aminobenzoic acid lotion or alcohol such as Lotion BPC containing EP-2 extract qs, Glycerol 20%, Alcohol 95% 60% Distilled water to 100% with or without Cetomacrogol emulsifying wax and/or Liquid paraffin and Glycerol, formulated in a fashion that will be familiar to those skilled in the art or as described in Hunting, 2004 and Remington, 2009. The pH and exact concentration of the various components of the composition are adjusted according to routine skills in the art.
- a gel for use in the treatment of proliferative skin disorders including scarring, pre-cancerous conditions and skin cancer, comprising EP-2 qs together with any one of the following: poloxamer gel 8E, poloxamer gel 8C, chlorhexidine gel or Lutrol® F127; formulated in a fashion that will be familiar to those skilled in the art or as described in Hunting, 2003; 2004 and Remington, 2009. The pH and exact concentration of the various components of the composition are adjusted according to routine skills in the art.
- an ointment for use in the treatment of proliferative skin disorders including scarring, pre-cancerous conditions and skin cancer (APF), comprising EP-2 qs, emulsifying wax, 30% and liquid paraffin or white soft paraffin 50% formulated in a fashion that will be familiar to those skilled in the art or as described in Remington, 2009. The pH and exact concentration of the various components of the composition are adjusted according to routine skills in the art.
- an Aqueous Cream APF for use in the treatment of proliferative skin disorders including scarring, pre-cancerous conditions and skin cancer, comprising EP-2 qs, Emulsifying ointment 30%, Glycerol 5%, Phenoxyethanol 1%, Vitamin E, 5% and water to 100%, formulated in a fashion that will be familiar to those skilled in the art or as described in Hunting, 2004 and Remington, 2009. The pH and exact concentration of the various components of the composition are adjusted according to routine skills in the art.

The composition may be adapted for personal care topical applications for use in the treatment or prevention of proliferative skin disorders including pre-cancerous conditions and skin cancers. For example, the composition may be used by subjects who have a proliferative skin disorder such as a pre cancerous condition and wish to treat it or who do not have cancer but wish to prevent the occurrence of cancer. For subjects wishing to treat or prevent a skin disorder such as psoriasis, eczema, scarring and pre-cancerous conditions, it is disclosed that the EP-2 may be formulated into a composition adapted for cosmetic skin care, such as sun burn cream, gels lotion, makeup preparations, lotions, creams, sticks, roll-ons formulations, mousses, aerosol sprays, pad-applied formulations, and film-forming formulations as described by Hunting, 2003; 2004.

In another form the composition adapted for personal care applications may be a cosmetic to treat a pre-cancerous condition. For example, the composition has the properties of treating or preventing the development of cancerous cells and also has a cosmetic property. The cosmetic composition may be in any form such as lotions, creams, sticks, roll-ons formulations, mousses, aerosol sprays, pad-applied formulations, and film-forming formulations, as described by Hunting, 2003; 2004.

In another example, the formulation may be used in subjects recovering from trauma of the skin due to injury or surgery who wish to minimize the scarring event that arises from such trauma, particularly those subjects susceptible to keloid scarring.

The composition may further comprise a second agent for use in the treatment and/or prevention of the proliferative skin disorder, pre-cancer or cancer. For example, the composition may additionally comprise a chemotherapeutic agent, an antibody or immunomodulatory agent directed at the over-proliferating cells. For example, if the proliferative disorder is a cancerous condition, the composition may also comprise dacarbazine, temozolomide, paclitaxel, cisplatin, carmustine, fotemustine, vindesine, vincristine, bleomycin, imiquimod or 5-Fluorouracil or combinations thereof. Alternative compounds, chemopreventive or chemotherapeutic agents known to the skilled addressee may also be provided in the compositions of the present invention.

It is disclosed that the avian extract EP-2 comprises about 50% to 95% by weight of the composition administered to the subject having the proliferative skin disorder, pre-cancer or cancer. For example, the composition may comprise at least 55%, 60%, 70% 80% or 90% EP-2. It is contemplated that in some instances the composition will comprise 100% EP-2, i.e. the avian extract will be administered directly to the subject as a neat extract. However, the exact amount of EP-2 in the composition will of course, depend on the route of delivery, the nature of the skin disorder, pre-cancer or cancer, the therapeutically effective amount of EP-2 required and the general nature and health of the subject to whom the composition is delivered. In some cases, the amount of EP-2 in a composition may be much lower, for example 1-50% EP-2 in the composition. It may be less than 50%, for example between 0.001% and 1.0%, up to 10%, 20%, 30%, 40% or 45% of the composition.

Examples of suitable formulations that are disclosed include: a formulation comprising 75% volume 95% ethanol and 25% EP-2 for topical application to the site of the proliferative skin disorder; a formulation comprising 25% volume DMSO and 75% EP-2 for topical application to the site of the proliferative skin disorder; or a formulation comprising 50% volume sterile buffered saline solution of less than 2% saline and 50% EP-2 for delivery internally through the skin surface into the site of a cancerous tumour or by injection into a tumour located internally.

It is disclosed that the concentration of the avian extract EP-2 will be of sufficient strength (qs) to provide a therapeutically effective dose to the target cells. Such therapy may be directed to reversibly stopping the growth of the abnormally proliferating cells, be they skin cells, cancer cells or pre-cancer cells. Alternatively, the therapeutically effective dose may be directed to stopping the growth of the cells irreversibly.

It is disclosed that the compositions comprising EP-2, may be adapted to be delivered to the subject in need once daily or less often. For example, if the composition is adapted for topical delivery, the composition may be administered to the skin once a day. However, it is contemplated that the composition can be administered more frequently than this (for example, two or three times a day) or less often (for example once a week). Alternatively, if the composition comprising EP-2 is in the form of an injectable formulation, it may be that the composition may be adapted for delivery once a week, or once a month.

The use of avian extract EP-2 in a therapeutically effective concentration together with a pharmaceutically acceptable carrier and/or diluent in the manufacture of composition for use in the treatment or prevention of proliferative skin conditions, cancers or pre-cancers is disclosed. The avian extract EP-2 may be isolated from tail feathers of a sulphur-crested cockatoo (Cacatua galerita). The avian extract EP-2 may be a synthetic mixture comprising: nicotinamide 1-10%, 2,4-ditert butyl phenol 1-10%, palmitic acid 5-40%, oleic acid 2-20%, and/or squalene 5-30%.

A kit comprising a composition comprising EP-2 and instructions regarding the administration of the composition for use in the treatment or prevention of a proliferative skin condition, pre-cancer or cancer is disclosed.

It is also disclosed a kit comprising a composition comprising EP-2 formulated for topical delivery and instructions regarding the topical application of the composition for use in the treatment of a proliferative skin condition. It is further disclosed that the kit comprises a composition comprising EP-2 formulated for topical delivery and instructions regarding the topical application of the composition for use in the treatment of a proliferative skin condition chosen from psoriasis, eczema or scarring, or a composition comprising EP-2 formulated for topical delivery and instructions regarding the topical application of the composition for the treatment of a proliferative skin condition chosen from skin cancer and pre-cancerous skin conditions.

There is also disclosed a kit comprising a composition comprising EP-2 formulated for parenteral delivery and instructions regarding the parenteral delivery of the composition for use in the treatment of a proliferative skin condition, pre-cancer or cancer. Further, the kit comprises a composition comprising EP-2 formulated for parenteral delivery and instructions regarding the parenteral delivery of the composition for use in the treatment of a proliferative skin condition chosen from psoriasis, eczema or scarring; a composition comprising EP-2 formulated for parenteral delivery and instructions regarding the parenteral delivery of the composition for the treatment of a proliferative skin condition chosen from skin cancer and pre-cancerous skin conditions; or a composition comprising EP-2 formulated for parenteral delivery and instructions regarding the parenteral delivery of the composition for the treatment of a proliferative condition chosen from cancer and pre-cancer.

The kits which are disclosed may comprise avian extract EP-2 isolated from tail feathers of a sulphur-crested cockatoo (Cacatua galerita) or alternatively avian extract EP-2 that is a synthetic mixture comprising: nicotinamide 1-10%, 2,4-ditert butyl phenol 1-10%, palmitic acid 5-40%, oleic acid 2-20%, and/or squalene 5-30%.

### Examples

The following examples are provided.

### Example 1

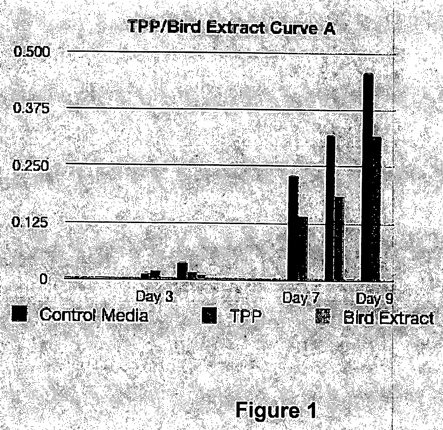

Saturated solutions of bird extract EP-2, 10 ng/mL triphenyl phosphate (TPP) or control media were used to saturate growth medium containing melanoma cells (A2058 Melanoma cells) at a concentration of approximately 1.2x104 cells/well or 1.0 x104 cells/well. All results are average cell counts for experiments performed in triplicate (see Figure 1). TPP was investigated on the basis of the findings in Zurhaar WO2008/019452 "Insect Repellent" that the insect repellent factor in an avian extract was TPP.

By day 3, the number of cells per well in both the control wells and wells containing TPP had doubled to approximately 2.5x104 cells/well. In contrast, wells containing bird extract EP-2 had only marginally increased in number relative to day 1.

By day 7, whereas both the TPP containing wells and control wells had increased in cell number relative to day 3, with the control wells containing approximately 20x104 cells/well and the TPP wells containing approximately 12.5 x 104 cells/well, the wells containing bird extract had no further cell growth relative to day 1.

By day 9, both the TPP containing wells and control wells had increased in cell number relative to day 7, with the control wells containing approximately 40.0 x 104 cells/well, and the TPP cells containing approximately 30.0 x 10 cells/well. In contrast, the cells containing bird extract had no further cell growth relative to day 1.

This represents a difference in growth rate of approximately 90% between wells containing avian extract EP-2 and those containing control medium or medium with TPP. Cells containing TPP showed a 25% lower growth rate than cells containing control medium by day 9 of the experiment.

Growth/Cell division appeared to be affected by the avian extract EP-2 but not by the TPP, indicating that either more than one component of the avian extract (EP-2) was having an effect on the cell growth or that TPP is not the agent responsible for the effect on cell growth.

### Example 2:

Pooled solvent vehicle (ethanol, methanol and pentane - used for EP-2 extraction), avian extract EP-2 and TPP were applied to 4x104 A2058 cells in 12 well plates.

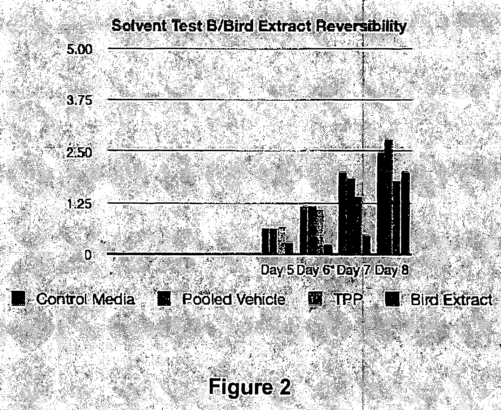

Figure 2 demonstrates that the pooled solvent vehicle did not appear to have any effect on the growth of cells compared to cells grown in control media alone, with both wells having cell counts of 2.5x106 cells/well at day 8. TPP had a moderate effect on the cells with cell counts of approximately 1.5 x 106 cells/well at day 8. The avian extract EP-2 had a pronounced effect on cell growth and division with cells at day 6 containing only slightly more cells than at the start of the experiment at day 0.

In order to determine if the effect of EP-2 on cell growth was reversible, on day 6 the avian extract EP-2 was removed from the cells by washing with Phosphate Buffered Saline and avian extract EP-2 saturated media was replaced with control media. It can be seen in Figure 2 that avian extract EP-2 had no toxic effect on cells and that removal of the extract lead to restoration of the normal growth rate and cell division by day 8.

This result indicates that the effect of the avian extract EP-2 on cells at low concentration is not permanent and appears to inhibit cell division rather than kill cells altogether, i.e. provides a 'static' effect.

At higher concentrations the effects are magnified and result in the permanent 'cidal' effects on the cells.

### Example 3

To further investigate the effect of EP-2 on cells in general, a 1/5 dilution of avian extract EP-2 was placed on 1 x 104 A2058 melanoma cells and HEK293 non-cancerous human embryonic kidney cells. A more dilute solution of avian extract EP-2 was used because non-cancerous embryonic cells do not have the same growth rate as cancer/melanoma cells so a reduced effect was expected.

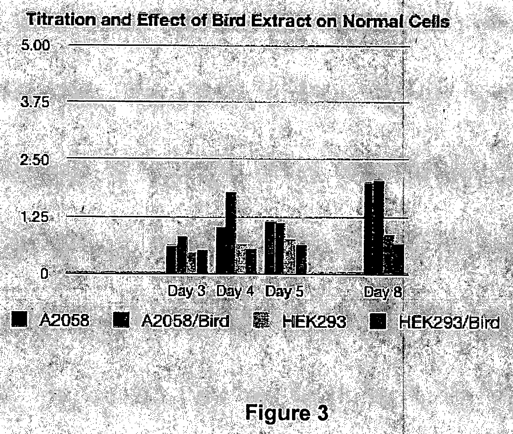

Figure 3 shows that the A2058 melanoma cells, being a fast growing cell line, were not as affected by EP-2 at 1/5 dilution as they had previously been shown to be by the concentrated extract (Examples 1 and 2). By day 8 the melanoma cells grown in dilute avian extract EP-2 had similar numbers of cells to control cells grown in normal cell culture medium.

The slower growing non-cancerous HEK293 cells were moderately affected by dilute avian extract EP-2 with respect to growth of cells and noticeably affected in cell morphology (results not shown).

### Example 4:

Avian extract EP-2 was applied to well developed sun keratoses (sun spots) on the forearms of a mature male subject. The application was made by rubbing ethanol extracted EP-2 in an oil base once daily on the affected region. Within a period of five days the sun spots were observed to slough off as a dried skin, leaving behind a mild pink region. The pink region faded and disappeared within seven days of the initial application.

### Example 5:

### Materials and Methods

### • Bird Extract (BE) media

Bird Extract (BE) (between 0.02 and 0.06g) was mixed with Dulbecco's Modified Eagle's Medium with high glucose, 4 mM L-glutamine and sodium pyruvate (DMEM) (Invitrogen). Medium plus BE was filtered.

### • Cell culture and reagents

A2058 Metastatic Melanoma cells, HEK293 (Human Embryonic Kidney cells) and Breast Cancer Cells (MCF-7) (ATCC) were cultured in Dulbecco's Modified Eagle's Medium with high glucose, 4 mM L-glutamine and sodium pyruvate (DMEM) (Invitrogen) supplemented with 10% FBS (fetal bovine calf serum) (Borogen Biologicals). Cells were plated in triplicate in 12-well plates at concentrations of 0.1x106 cells/ml. Cell cultures were incubated at 37°C in a humidified incubator, supplemented with 5% CO2. Cells were dissociated with 5mM EDTA in DMEM.

### • Cell counts (Vi-Cell)

The cells/ml were counted before, during and after treatment using Vi-cell-XR cell viability analyzer (Beckman Coulter).

### • Haematoxylin & Eosin staining to assess cell morphology and membrane integrity

Mayer's Haematoxylin & Eosin (0,5 % eosin) staining was performed on Melanoma cells to show morphological changes after BE treatment. Approximately 5 × 10 4 cells per cover slip were seeded and fixed with 4% paraformaldehyde for 10 minutes and washed twice with PBS (Phosphate Buffered Saline).

Stained cells were assessed using a BX51 microscope and photos were taken with an OLYMPUS DP71 camera and photos.

### • Nuclear Morphology, DAPI staining

Staining of the nucleus of melanoma cells was carried out with ProLong Gold anti-fade reagent containing DAPI (Invitrogen) for detecting nuclear integrity, and whether apoptosis is induced (Soto et al. 2003) after treatment with BE. Approximately 5 × 10 4 cells per cover slip were seeded and fixed with 1% paraformaldehyde for 15 minutes. Cells were stained with DAPI and were visualized through an OLYMPUS BX51 microscope with the exposure 1/9 seconds and photos were taken with an OLYMPUS DP71 fluorescent camera.

### • Cytotoxicity

The cytotoxicity was assessed by the CytoTox-ONE™ Homogeneous Membrane Integrity Assay from Promega by measuring the amount of LDH in the supernatant after treating Melanoma cells and Embryonic Kidney cells with the BE in a 96-well plate. The maximum amount of LDH from both cell lines was determined by the addition of lysis solution (9% Triton® X-100 in water). A microplate reader (FLUOstar OPTIMA) from BMG Labtechnologies was used to measure the fluorescence. The excitation wavelength was set at 544 nm and emission at 590 nm.

Cells were cultured in 96-well plates and Melanoma A2058 cells were plated at a density of 2 x 103 cells/well and HEK293 cells at 1.8x103 cells /well in a final volume of 100 µL of complete medium. In order to reduce the background fluorescence absorbance, phenol-red-free DMEM was used (supplemented with reduced serum of 1%).

### • RNA extraction and RT-qPCR

Differences in gene expression between cells grown in the presence and absence of BE were determined by quantitative RT-PCR analysis. Total RNA was extracted from cultured A2058 Melanoma cells using RNA extraction kits (Qiagen) according to the manufacturer's recommendations. Quantity and quality of the isolated RNA was measured using the Agilent RNA 6000 Nano Kit with an Agilent 2100 bioanalyser. 250ng of total RNA was reverse transcribed using Omniscript RT kit (Qiagen) and PCR products were amplified with KAPA SYBR FAST qPCR Master Mix (KapaBiosystems), for 40 cycles. Primer design was established with the aid of the online resource, National Center for Biotechnology Information (NCBI). Each PCR reaction was performed in triplicate and the mean Ct value was used to calculate the fold change over GAPDH (the house keeping gene) using the ΔΔCt method.

PCR sizes were confirmed with gel electrophoresis and visualised on a UV gel documentation system by comparing with a 100 bp Ladder DNA marker (100-3,000 bp) from Axygen.

### • Statistical analysis

Statistical analysis of gene expression (RT-qPCR) was performed using the Student's t-test.

Statistical analysis of the number of cells with and without treatment was performed using the Student's t-test.

### Results

### • Effects of Bird Extract on cell proliferation

Incubation of Melanoma Cells with Bird Extract (BE) for 1, 2 and 3 days showed a dramatic loss of cells relative to cells grown in the absence of Bird Extract (BE) (Figures 1a and 1b and Table 1).

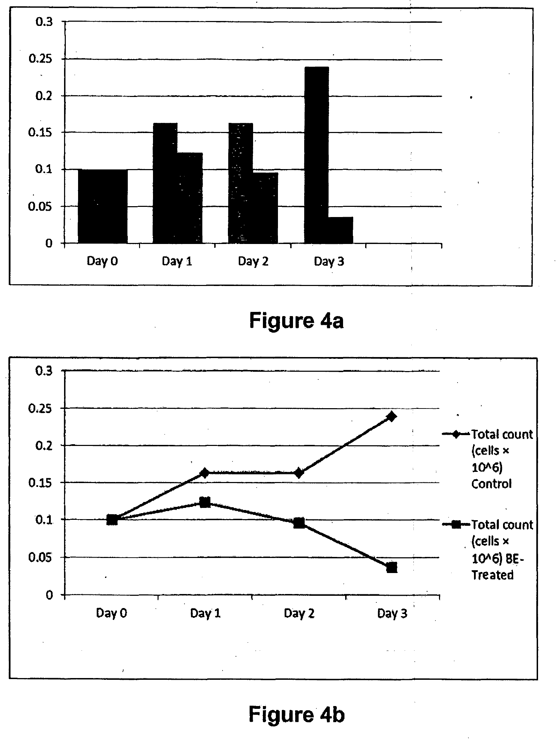

Cells grown in the presence of Bird Extract showed a 70% loss of cell numbers by day 3 whereas control untreated cells increased cell numbers by 70% over 3 days (Figures 4a and 4b, Table 1).

**TABLE 1: Number of Melanoma cells (× 10^6) grown in the presence or absence of Bird Extract (BE) for 1-3 days**

| | **Sample Number** | **Cell Count (x 10^6/ml)** | **Average Cell Count (x 10^6/ml)** | **Std Dev (x 10^6/ml)** |
|---|---|---|---|---|
| **DAY0** | | 0.1 | 0.1 | - |

| **DAY 1** | | | | |
|---|---|---|---|---|
| Control | 1 | 0.18 | | |
| | 2 | 0.17 | 0.163 | 0.021 |
| | 3 | 0.14 | | |
| | | | | |
| BE-treated | 1 | 0.12 | | |
| | 2 | 0.11 | 0.123 | 0.026 |
| | 3 | 0.14 | | |

| **DAY 2** | | | | |
|---|---|---|---|---|
| Control | 1 | 0.17 | | |
| | 2 | 0.16 | 0.163 | 0.010 |
| | 3 | 0.16 | | |
| BE-treated | 1 | 0.087 | | |
| | 2 | 0.1 | 0.096 | 0.013 |
| | 3 | 0.1 | | |

| **DAY 3** | | | | |
|---|---|---|---|---|
| Control | 1 | 0.26 | | |
| | 2 | 0.2 | 0.24 | 0.035 |
| | 3 | 0.26 | | |
| BE-treated | 1 | 0.046 | | |
| | 2 | 0.024 | 0.037 | 0.02 |
| | 3 | 0.04 | | |

### • Effects of varying concentrations of Bird Extract on cell proliferation and survival

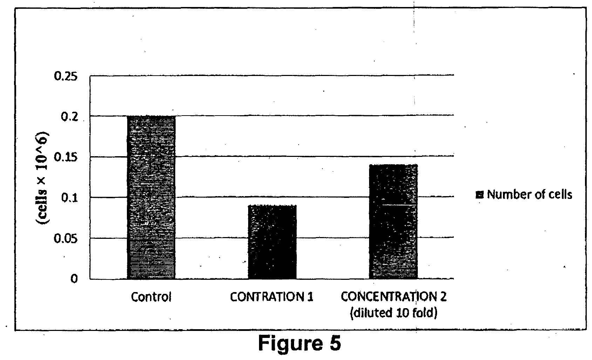

Results indicate that increasing the concentration of Bird Extract (BE) increases the effect on cell proliferation: ie increasing the amount of BE in the cell medium reduces cell proliferation and increases cell death in Melanoma cells. Therefore, higher concentrations of BE are more effective at causing cell death and inhibition of cell proliferation than low concentrations (Figure 5, Table 2).

**TABLE 2: Number of Melanoma A2058 cells after 1 days growth at zero (control) or various concentrations of BE.**

| | **Sample number** | **Total cell count (× 10^6 /ml)** | **Average (cell count × 10^6 /ml)** | **Std Dev (× 10^6/ml)** |
|---|---|---|---|---|
| **DAY 0** | 1 | 0.1 | 0.1 | - |
| | | | | |

| **DAY 1** | | | | |
|---|---|---|---|---|
| Control | 1 | 0.28 | | |
| | 2 | 0.17 | 0.20 | 0.07 |
| | 3 | 0.15 | | |
| C1 (high) | 1 | 0.091 | | |
| | 2 | 0.099 | 0.092 | 0.01 |
| | 3 | 0.087 | | |
| C2 (1/10 dilution) | 1 | 0.13 | | |
| | 2 | 0.17 | 0.14 | 0.03 |
| | 3 | 0.12 | | |

### • Effect of Bird Extract on cell proliferation in melanoma and breast cancer cells

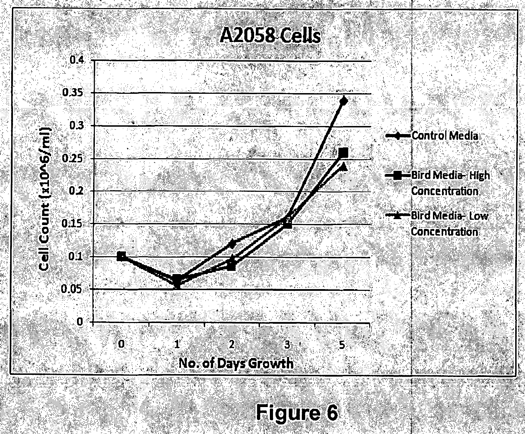

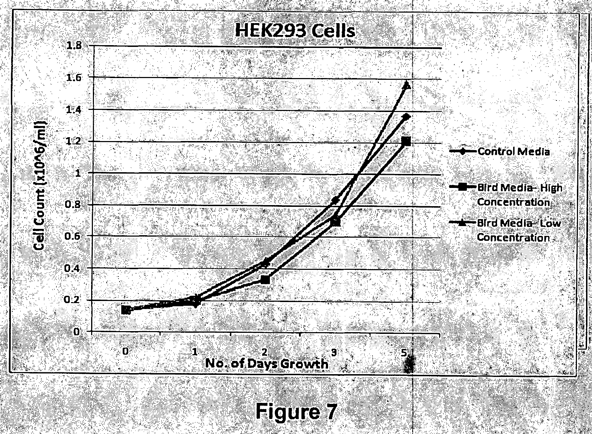

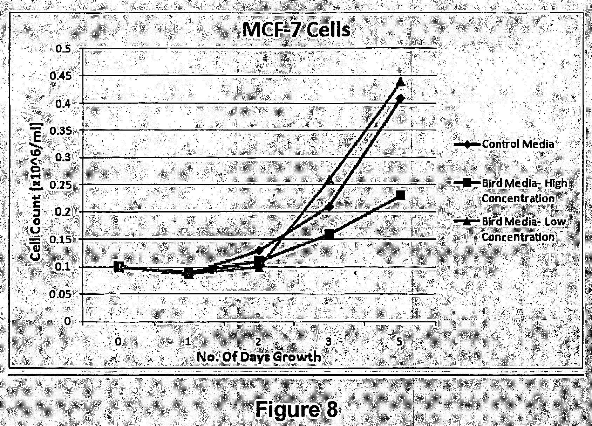

Results indicate that Bird Extract (BE) reduces cell proliferation in Melanoma cells and in Breast Cancer cells to a greater extent than in non-cancerous cells (Embryonic Kidney (HEK 293)) cell lines (Figures 6, 7 and 8, Tables 3A-C).

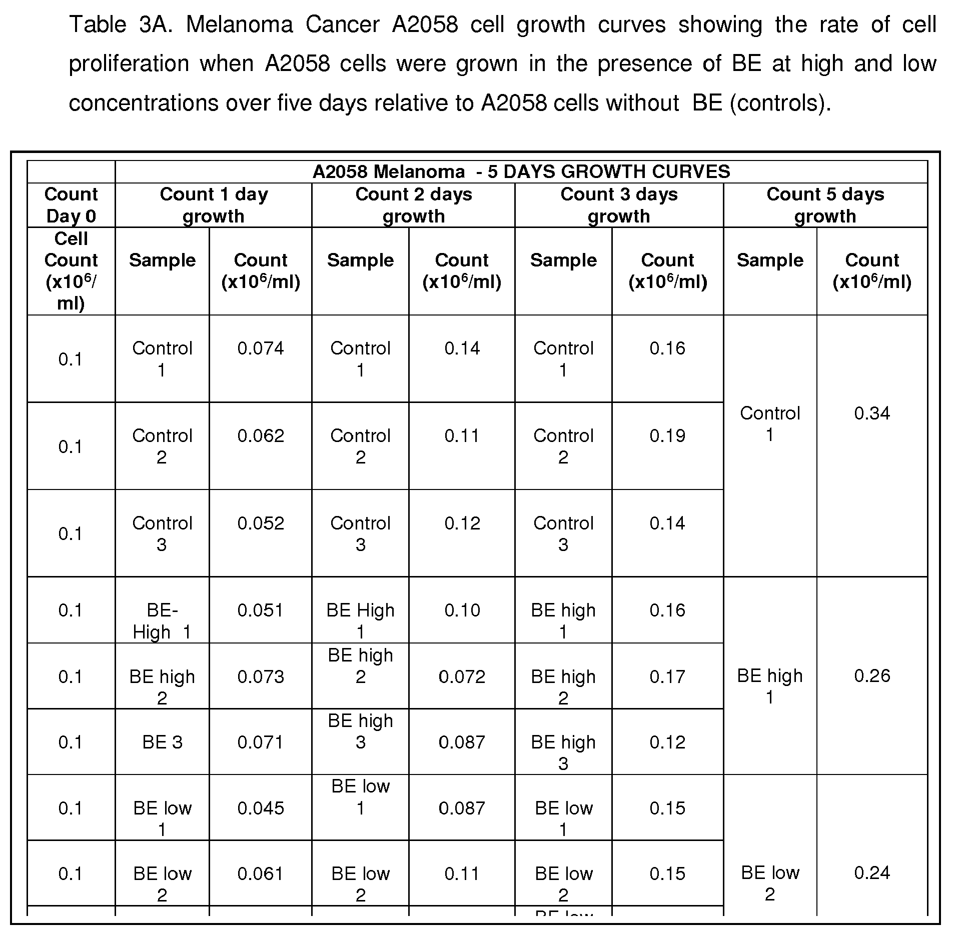

**Table 3B: Non-cancerous Human Embryonic Kidney (HEK293) cellular growth curves showing the rate of cell proliferation when HEK293 cells were grown in the presence of BE at high and low concentrations over five days relative to HEK293 cells without BE (controls).**

| | **HEK293 Cells** | | | | | | | |
|---|---|---|---|---|---|---|---|---|
| **Count Day 0** | **Count 1 day growth** | | **Count 2 days growth** | | **Count 3 days growth** | | **Count 5 days growth** | |
| **Cell Count (x10⁶/ml)** | **Sample** | **Count (x10⁶/ml)** | **Sampl e** | **Count (x10⁶/ml)** | **Sample** | **Count (x10⁶/ml)** | **Sample** | **Count (x10⁶/ml)** |
| 0.1 | Control 1 | 0.17 | Control 1 | 0.44 | Control 1 | 0.63 | Control 1 | 1.37 |
| 0.1 | Control 2 | 0.19 | Control 2 | 0.48 | Control 2 | 1.15 | | |
| 0.1 | Control 3 | 0.19 | Control 3 | 0.38 | Control 3 | 0.73 | | |
| 0.1 | BE high 1 | 0.21 | BE high 1 | 0.33 | BE high 1 | 0.71 | BE high 1 | 1.21 |
| 0.1 | BE high 2 | 0.21 | BE high 2 | 0.33 | BE high 2 | 0.71 | | |
| 0.1 | BE high 3 | 0.18 | BE high 3 | 0.34 | BE high 3 | 0.69 | | |
| 0.1 | BE low 1 | 0.18 | BE low 1 | 0.41 | BE low 1 | 0.65 | BE low 1 | 1.57 |
| 0.1 | BE low 2 | 0.25 | BE low 2 | 0.44 | BE low 2 | 1.00 | | |
| 0.1 | BE low 3 | 0.22 | BE low 3 | 0.49 | BE low 3 | 0.56 | | |

**Table 3C: Breast Cancer cell (MCF-7) growth curves showing the rate of cell proliferation when Breast cancer cells were grown in the presence of BE at high and low concentrations over five days relative to Breast Cancer cells without BE (controls).**

| | **MCF (Breast Cancer) Cells** | | | | | | | |
|---|---|---|---|---|---|---|---|---|
| **Count Day 0** | **Count 1- 1 day growth** | | **Count 2- 2 days growth** | | **Count 3- 3 days growth** | | **Count 4- 5 days growth** | |
| **Count (x10⁶/ml)** | **Sample** | **Count (x10⁶/ml)** | **Sample** | **Count (x10⁶/ml)** | **Sample** | **Count (x10⁶/ml)** | **Sample** | **Count (x10⁶/ml)** |
| 0.1 | Control 1 | 0.095 | Control 1 | 0.15 | Control 1 | 0.18 | Control 1 | 0.41 |
| 0.1 | Control 2 | 0.083 | Control 2 | 0.14 | Control 2 | 0.23 | | |
| 0.1 | Control 3 | 0.084 | Control 3 | 0.11 | Control 3 | 0.23 | | |
| 0.1 | BE high 1 | 0.079 | BE high 1 | 0.10 | BE high 1 | 0.15 | BE high 1 | 0.23 |
| 0.1 | BE high 2 | 0.10 | BE high 2 | 0.13 | BE high 2 | 0.17 | | |
| 0.1 | BE high 3 | 0.088 | BE high 3 | 0.11 | BE high 3 | 0.15 | | |
| 0.1 | BE low 1 | 0.082 | BE low 1 | 0.11 | BE low 1 | 0.25 | BE low 1 | 0.44 |
| 0.1 | BE low 2 | 0.078 | BE low 2 | 0.10 | BE low 2 | 0.31 | | |
| 0.1 | BE low 3 | 0.10 | BE low 3 | 0.094 | BE low 3 | 0.22 | | |

### • Effect of Bird Extract on cell morphology, cell membrane integrity and nuclear morphology indicative of apoptosis

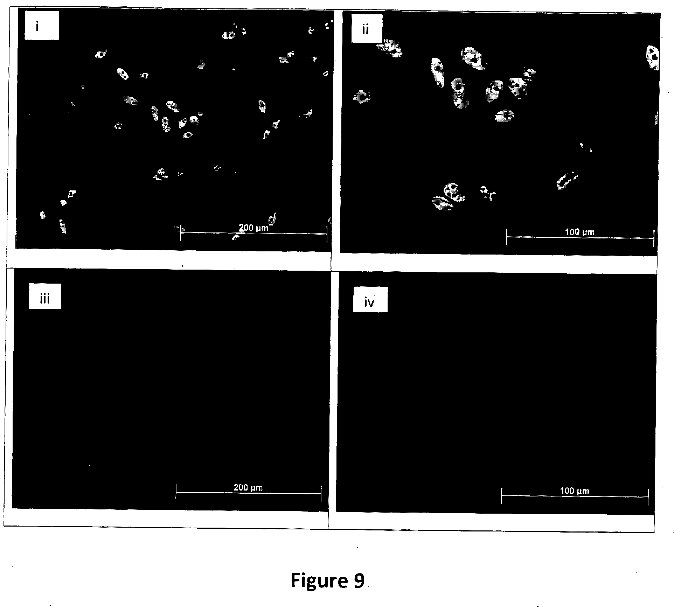

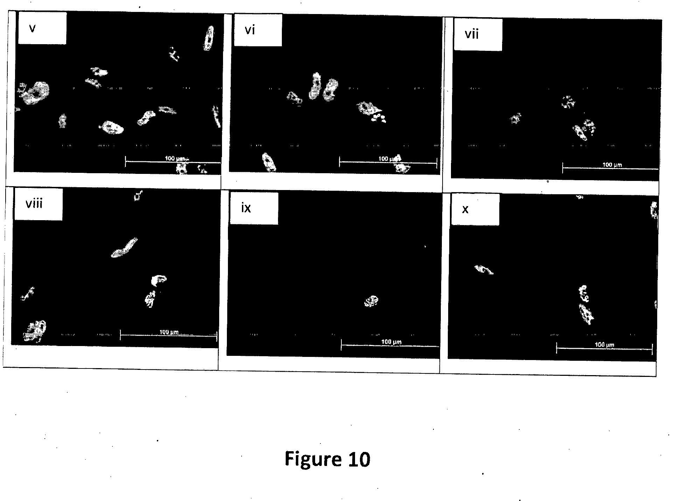

Results indicate that Bird Extract causes cells to become enlarged and rounded in shape with altered nuclear structures indicative of apoptosis (programmed cell death). Cells containing Bird Extract contain dark, condensed nucleoli amid bright perinuclear activity. Untreated cells remain elongated with multiple euchromatic nucleoli and lower perinuclear activity (Figure 9 i-iv and Figure 10).

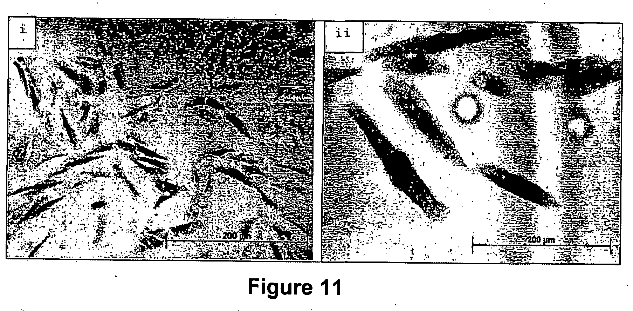

Haemotoxylin and Eosin stain (Figure 11) shows that cells incubated with Bird Extract have altered morphology, and condensed nuclei, with very darkly stained chromatin, as well as severely affected nuclear membranes. There are very large perinuclear structures, possibly sites of lipid accumulation as observed for glycogen deposits (Jiang et al., 2010) or swollen mitochondria.

### • Toxicity testing

Cell death can occur either by apoptosis or by necrosis. Necrosis is accompanied by mitochondrial swelling and increased plasma membrane permeability, whereas apoptosis involves an articulated breakdown of the cell into membrane-bound apoptotic bodies. Lactate dehydrogenase (LDH) is a soluble cytosolic enzyme that is released into the culture medium following loss of membrane integrity resulting from either apoptosis or necrosis. LDH activity, therefore, can be used as an indicator of cell membrane integrity and serves as a general means to assess cytotoxicity resulting from chemical compounds or environmental toxic factors.

Therapeutic agents are often tested for their cytotoxic effect on cancer cells with the aim of finding a compound that causes cell death by toxic effect on cells (Casado-Zapico [2010] Journal of Pineal Research 48(1): 72-80). The cell death may be caused by a disruption of the cell membrane integrity evoked by either apoptosis or necrosis.

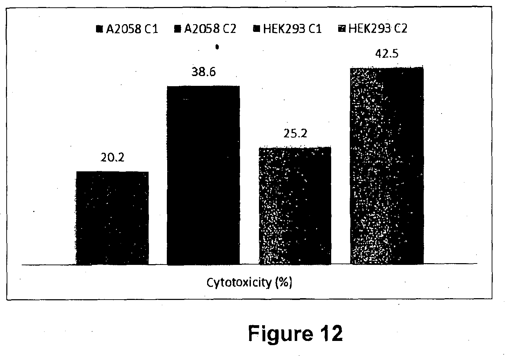

Results of the toxicity tests indicate that the Bird Extract (BE) is cytotoxic to both Melanoma cells (A2058) and non-cancerous cells (Embryonic Kidney (HEK 293)) cell lines to a similar extent. The concentration of the BE in the cell culture medium determines the level of cytotoxity. Higher concentrations (C2) of BE cause the release of more LDH and are therefore more toxic than lower concentrations of BE (C1) relative to controls (untreated cells) (Figure 12).

### • Gene expression studies

The effect of Bird Extract was assessed on expression levels of genes involved in pathways of cell survival and proliferation leading to apoptosis (Maryann et al. [2005] Clinical cancer research 11 (14): 5153-5157). By targeting certain pathways in the PTEN-pathway, either by PI3K, AKT or mTOR, apoptotic signals could potentially be triggered. MITF may also be differentially expressed as a result of changes to PTEN levels (Garraway [2005] Nature (London) 436(7047): 117-122). Here we assessed the gene expression levels of several genes known to be associated with cell proliferation and apoptosis (Rose Boutros [2004] Biochemical and Biophysical Research Communications 325(4): 1115-1121).

Genes analysed for expression levels by quantitative RT-PCR include:
- PTEN
- CCNA2
- TPD52
- Bcl-XL
- TGF- β

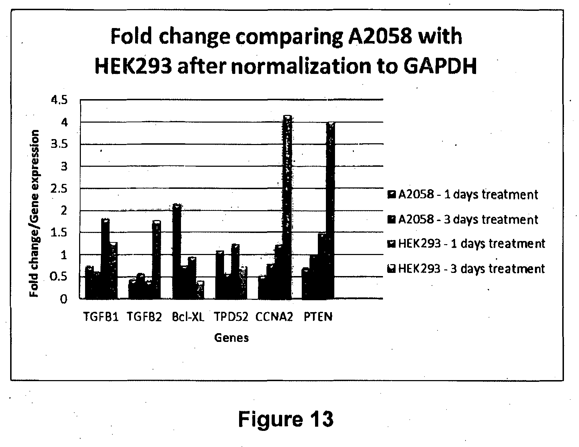

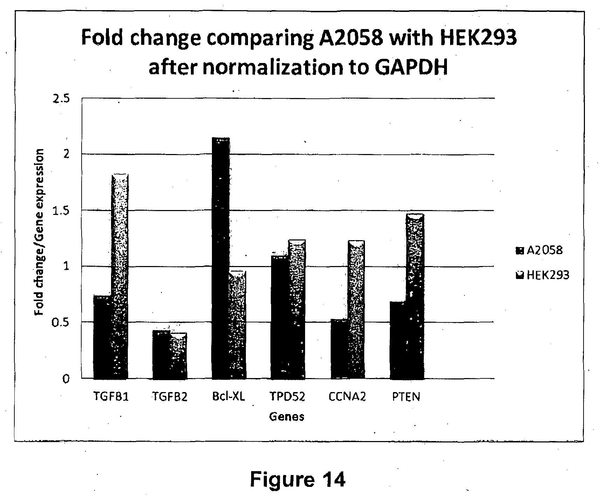

Both Melanoma cells and Embryonic Kidney cells show changes in gene expression patterns after treatment with Bird Extract (Figures 13 and 14), particularly in Kidney cells where both CCNA2 and PTEN showed 4 fold changes in gene expression levels. Moreover Melanoma cells showed a two-fold change in BclXI levels. These changes are indicative of cells undergoing apoptosis, confirming that the Bird Extract causes significant cell death in cells in culture.

### Example 6

Results above indicate that Bird Extract (BE) inhibits cell proliferation in Melanoma cells and in Breast Cancer cells as well as in non-cancerous (Embryonic Kidney (HEK 293)) cell lines (Figures 6, 7 and 8, Tables 3A-C). This result indicates that the Bird Extract is toxic to all cells.

In an attempt to reduce the non-specific toxicity and to ensure that results were more consistent, a Synthetic Mixture was formulated, based on an analysis of the components of the Bird Extract which had dissolved in cell culture media. The Synthetic Mixture comprised the following:

| **Compound** | **%** | **mg/30mL** | **mg/L** |
|---|---|---|---|
| Nicotinamide | 5.89 | 1.77 | 58.9 |
| 2,4-ditert butyl phenol | 2.44 | 0.73 | 24.4 |
| Palmitic acid | 19.76 | 5.93 | 197.6 |
| Oleic acid | 7.57 | 2.27 | 75.7 |
| Squalene | 12.06 | 3.62 | 120.6 |

The Synthetic Mixture may optionally also include BHT-aldehyde (0.22%, 2.2mg/L) and/or cholestenol (4.02%, 40.2mg/L).

The Synthetic Mixture can be dissolved in methanol as the solvent, or in water as the solvent to generate a solution for use as an extract.

The Synthetic Mixture (0.5g) was mixed with Dulbecco's Modified Eagle's Medium with high glucose, 4 mM L-glutamine and sodium pyruvate (DMEM) (Invitrogen), and then filtered.

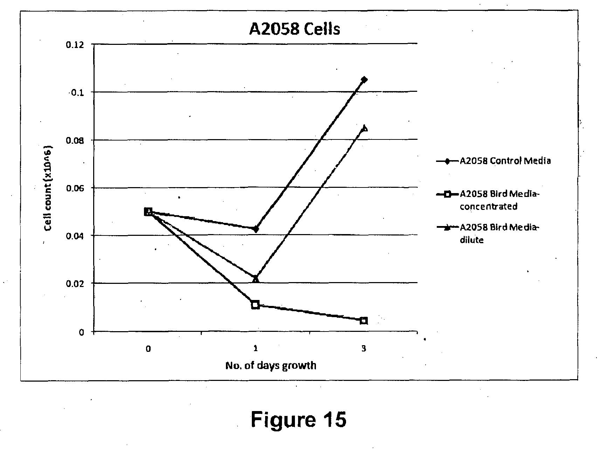

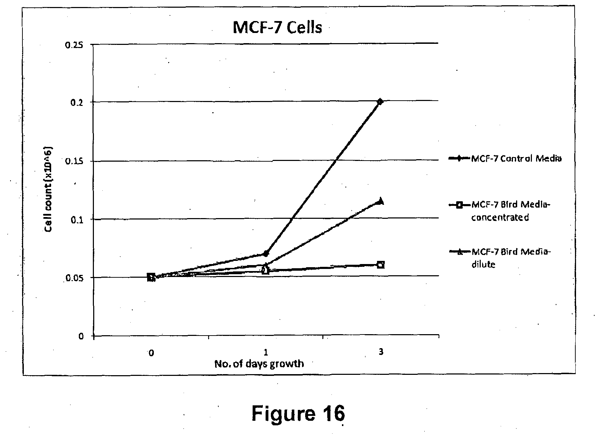

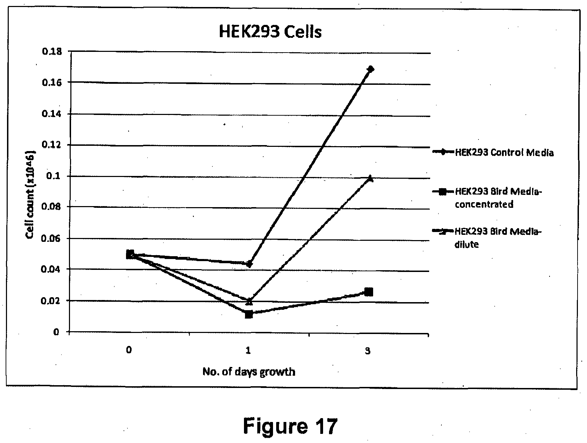

The Synthetic Mixture was dissolved in cell culture media at a high (C1) and a low concentration (C2). Melanoma cells (A2058), Breast cancer cells (MCF-7) and Embryonic Kidney cells (HEK293) were then grown in cell culture media plus Synthetic Extract for three days (Figures 15, 16 and 17).

The exposure of the cells to the Synthetic Mixture shows that all the cell types are dramatically affected by the Synthetic Mixture, particularly at high concentrations. Melanoma cells appear to be the most severely affected at high concentrations with the majority of the cells undergoing cell death by day 3. This shows that the Synthetic Mixture is highly effective at destroying the A2058 Melanoma cells at high concentrations.

**Table 4: Melanoma Cancer (A2058) cells, Breast Cancer (MCF-7) cells and Embryonic Kidney (Hek293) cells exposed to Synthetic Mixture for 1 and 3 days. Cell proliferation rates are shown when cells were grown in the presence of Synthetic Mixture at high (C1) and low (C2) concentrations over one and three days relative to cells without Synthetic Mixture (controls).**

| | ***Count 1 (1 day growth)*** | | | | ***Count 2 (3 days growth)*** | | | |
|---|---|---|---|---|---|---|---|---|
| **Sample** | **Count (x10⁶/ml)** | **Mean Count** | **Viability (%)** | **Mean Viability** | **Count (x10⁶/ml)** | **Mean Count** | **Viability (%)** | **Mean Viability** |
| **A2058 control-1** | 0.074 | 0.085 | 93.4 | 93.7 | 0.23 | 0.21 | 87.3 | 87.1 |
| **A2058 control-2** | 0.085 | | 92.9 | | 0.20 | | 89.2 | |
| **A2058 control-3** | 0.095 | | 94.9 | | 0.19 | | 84.7 | |
| **A2058 bird C1-1** | 0.023 | 0.022 | 89.5 | 95.1 | 0.011 | 0.009 | 55.6 | 45.9 |
| **A2058 bird C1-2** | 0.028 | | 95.7 | | 0.006 | | 60.0 | |
| **A2058 bird C1-3** | 0.015 | | 100 | | 0.011 | | 22.2 | |
| **A2058 bird C2-1** | 0.039 | 0.044 | 90.6 | 90.6 | 0.17 | 0.17 | 67.9 | 76.4 |
| **A2058 bird C2-2** | 0.044 | | 86.1 | | 0.17 | | 80.9 | |
| **A2058 bird C2-3** | 0.049 | | 95.0 | | 0.16 | | 80.5 | |
| **HEK293 control-1** | 0.069 | 0.088 | 87.7 | 89.3 | 0.36 | 0.34 | 88.6 | 85.8 |
| **HEK293 control-2** | 0.096 | | 92.4 | | 0.32 | | 81.8 | |
| **HEK293 control-3** | 0.099 | | 87.7 | | 0.33 | | 87.1 | |
| **HEK293 bird C1-1** | 0.037 | 0.024 | 80.0 | 79.0 | 0.042 | 0.053 | 71.4 | 64.7 |
| **HEK293 bird C1-2** | 0.017 | | 71.4 | | 0.050 | | 59.2 | |
| **HEK293 bird C1-3** | 0.017 | | 85.7 | | 0.067 | | 63.6 | |
| **HEK293 bird C2-1** | 0.049 | 0.041 | 90.0 | 80.0 | 0.18 | 0.20 | 59.7 | 65.0 |
| **HEK293 bird C2-2** | 0.044 | | 83.3 | | 0.22 | | 65.5 | |
| **HEK293 bird C2-3** | 0.029 | | 66.7 | | 0.21 | | 69.7 | |
| **MCF-7 control-1** | 0.15 | 0.14 | 86.3 | 80.5 | 0.41 | 0.40 | 94.4 | 93.7 |
| **MCF-7 control-2** | 0.13 | | 81.7 | | 0.45 | | 94.0 | |
| **MCF-7 control-3** | 0.13 | | 73.6 | | 0.34 | | 92.6 | |
| **MCF-7 bird C1-1** | 0.12 | 0.11 | 68.3 | 68.5 | 0.13 | 0.12 | 91.4 | 89.3 |
| **MCF-7 bird C1-2** | 0.10 | | 72.6 | | 0.14 | | 88.6 | |
| **MCF-7 bird C1-3** | 0.10 | | 64.7 | | 0.10 | | 88.0 | |
| **MCF-7 bird C2-1** | 0.10 | 0.12 | 80.5 | 77.9 | 0.22 | 0.23 | 85.4 | 84.0 |
| **MCF-7 bird C2-2** | 0.11 | | 73.4 | | 0.25 | | 79.7 | |
| **MCF-7 bird C2-3** | 0.16 | | 79.7 | | 0.22 | | 87.0 | |

Numerous variations and modifications will suggest themselves to persons skilled in the relevant art, in addition to those already described, without departing from the basic inventive concepts.

## Claims

1. A composition comprising nicotinamide 1-10%, 2,4-ditert butyl phenol 1-10%, palmitic acid 5-40%, oleic acid 2-20%, and squalene 5-30% together with a pharmaceutically acceptable carrier and/or diluent for use in the treatment or prevention of cancers or pre-cancers selected from the group consisting of: skin cancer (including basal cell carcinoma, squamous carcinoma or melanoma), precancerous skin lesion (such as actinic or solar keratosis), carcinoma (including breast, prostate, lung, pancreatic and colon cancers), sarcoma, lymphoma, germ cell tumour, blastoma, and leukaemia.

2. The composition for use according to claim 1, wherein the composition comprises: nicotinamide at 5.9%, 2,4-ditert butyl phenol at 2.5%, palmitic acid at 19.8%, oleic acid at 7.6%, and squalene at 12.1%.

3. The composition for use according to claim 1 or 2, wherein the composition further comprises: BHT-aldehyde at 0.05-2% and/or cholestenol at 1-10%.

4. A kit for use in the treatment or prevention of a cancer or pre-cancer comprising: a composition comprising nicotinamide 1-10%, 2,4-ditert butyl phenol 1-10%, palmitic acid 5-40%, oleic acid 2-20%, and/or squalene 5-30% and a pharmaceutically acceptable carrier and/or diluent; and instructions regarding the administration of the composition for the treatment or prevention of a cancer or pre-cancer, wherein the cancer or pre-cancer is selected from the group consisting of: skin cancer (including basal cell carcinoma, squamous carcinoma or melanoma), precancerous skin lesion (such as actinic or solar keratosis), carcinoma (including breast, prostate, lung, pancreatic and colon cancers), sarcoma, lymphoma, germ cell tumour, blastoma, and leukaemia.

## Patentansprüche

1. Zusammensetzung, umfassend 1-10% Nicotinamid, 1-10% 2,4-Di-tert.-butylphenol, 5-40% Palmitinsäure, 2-20% Ölsäure und 5-30% Squalen zusammen mit einem pharmazeutisch unbedenklichen Träger und/oder Verdünnungsmittel zur Verwendung bei der Behandlung oder Prävention von Krebserkrankungen oder präkanzerösen Erkrankungen ausgewählt aus der Gruppe bestehend aus: Hautkrebs (einschließlich Basalzellenkarzinom, Plattenepithelkarzinom oder Melanom), präkanzerösen Hautläsionen (wie aktinischer oder solarer Keratose), Karzinomen (einschließlich Brust-, Prostata-, Lungen-, Bauchspeicheldrüsen- und Dickdarmkrebs), Sarkom, Lymphom, Keimzelltumor, Blastom und Leukämie.

2. Zusammensetzung zur Verwendung Anspruch 1, wobei die Zusammensetzung Folgendes umfasst: 5,9% Nicotinamid, 2,5% 2,4-Di-tert.-butylphenol, 19,8% Palmitinsäure, 7,6% Ölsäure und 12,1% Squalen.

3. Zusammensetzung zur Verwendung nach Anspruch 1 oder 2, wobei die Zusammensetzung weiterhin Folgendes umfasst: 0,05-2% BHT-Aldehyd und/oder 1-10% Cholestenol.

4. Kit zur Verwendung bei der Behandlung oder Prävention einer Krebserkrankung oder einer präkanzerösen Erkrankung, umfassend: eine Zusammensetzung, die 1-10% Nicotinamid, 1-10% 2,4-Di-tert.-butylphenol, 5-40% Palmitinsäure, 2-20% Ölsäure und/oder 5-30% Squalen und einen pharmazeutisch unbedenklichen Träger und/oder ein pharmazeutisch unbedenkliches Verdünnungsmittel umfasst, und Anweisungen zur Verabreichung der Zusammensetzung zur Behandlung oder Prävention einer Krebserkrankung oder einer präkanzerösen Erkrankung, wobei die Krebserkrankung bzw. die präkanzeröse Erkrankung ausgewählt ist aus der Gruppe bestehend aus: Hautkrebs (einschließlich Basalzellenkarzinom, Plattenepithelkarzinom oder Melanom), präkanzerösen Hautläsionen (wie aktinischer oder solarer Keratose), Karzinomen (einschließlich Brust-, Prostata-, Lungen-, Bauchspeicheldrüsen- und Dickdarmkrebs), Sarkom, Lymphom, Keimzelltumor, Blastom und Leukämie.

## Revendications

1. Composition comprenant du nicotinamide 1 à 10 %, du 2,4-di-tert-butyl-phénol 1 à 10 %, de l'acide palmitique 5 à 40 %, de l'acide oléique 2 à 20 %, et/ou du squalène 5 à 30 % conjointement avec un support et/ou un diluant pharmaceutiquement acceptable pour une utilisation dans le traitement ou la prévention de cancers ou de pré-cancers choisis dans le groupe constitué par : un cancer de la peau (y compris un carcinome basocellulaire, un carcinome squameux ou un mélanome), une lésion précancéreuse de la peau (telle qu'une kératose actinique ou solaire), un carcinome (y compris les cancers du sein, de la prostate, du poumon, pancréatique et du côlon), un sarcome, un lymphome, une tumeur germinale, un blastome et une leucémie.

2. Composition pour une utilisation selon la revendication 1, la composition comprenant : nicotinamide à raison de 5,9 %, du 2,4-di-tert-butyl-phénol à raison de 2,5 %, de l'acide palmitique à raison de 19,8 %, de l'acide oléique à raison de 7,6 %, et du squalène à raison de 12,1 %.

3. Composition pour une utilisation selon la revendication 1 ou 2, la composition comprenant en outre : aldéhyde-BHT à raison de 0,05 à 2 % et/ou du cholesténol à raison de 1 à 10 %.

4. Kit pour une utilisation dans le traitement ou la prévention d'un cancer ou d'un pré-cancer comprenant : une composition comprenant du nicotinamide 1 à 10 %, du 2,4-di-tert-butyl-phénol 1 à 10 %, de l'acide palmitique 5 à 40 %, de l'acide oléique 2 à 20 %, et/ou du squalène 5 à 30 % et un support et/ou un diluant pharmaceutiquement acceptable ; et des instructions concernant l'administration de la composition pour le traitement ou la prévention d'un cancer ou d'un pré-cancer, le cancer ou le pré-cancer étant choisi dans le groupe constitué par : un cancer de la peau (y compris un carcinome basocellulaire, un carcinome squameux ou un mélanome), une lésion précancéreuse de la peau (telle qu'une kératose actinique ou solaire), un carcinome (y compris les cancers du sein, de la prostate, du poumon, pancréatique et du côlon), un sarcome, un lymphome, une tumeur germinale, un blastome et une leucémie.
